# EUROPEAN PATENT SPECIFICATION

(11) **EP 1 333 817 B1**
(45) Date of publication and mention of the grant of the patent: **11.03.2015**
(21) Application number: 01986601.1
(22) Date of filing: 11.10.2001
(51) Int. Cl.: A61K 9/70

(54) **FILM FOR ACTIVE INGREDIENTS DERMAL AND TRANSDERMAL ADMINISTRATION**
FILM ZUR DERMALEN ODER TRANSDERMALEN VERABREICHUNG VON WIRKSTOFFE
FILM POUR L'ADMINISTRATION CUTANEE ET TRANSCUTANEE DE PRINCIPES ACTIFS

(30) Priority: 13.10.2000 IT MI20002216
(43) Date of publication of application: 13.08.2003
(73) Proprietor: LABORATORIO ITALIANO BIOCHIMICO FARMACEUTICO LISAPHARMA S.P.A., 22036 Erba (Como) (IT)
(72) Inventor: COLOMBO, Paolo, I-43100 Parma (IT); CATELLANI, Pier, Luigi, I-42100 Reggio Emilia (IT); PADULA, Cristina, I-85018 Trivigno (IT); SANTI, Patrizia, I-43039 Salsomaggiore Terme (IT); COLOMBO, Gaia, I-43100 Parma (IT)
(74) Representative: Gervasi, Gemma
(86) International application number: PCT/EP2001/011768
(87) International publication number: WO 2002/030402

(56) References cited:
- EP-A- 0 184 470
- EP-A- 0 355 536
- EP-A- 1 110 546
- WO-A-00/45795
- GB-A- 1 108 837
- GB-A- 2 188 844
- US-A- 4 210 633
- DATABASE WPI Section Ch, Week 198325 Derwent Publications Ltd., London, GB; Class A11, AN 1983-59663K XP002166687 & JP 58 078663 A (WATANABE YAKUHIN KOGYO KK), 12 May 1983 (1983-05-12)
- DATABASE WPI Section Ch, Week 198645 Derwent Publications Ltd., London, GB; Class A96, AN 1986-295725 XP002166688 & JP 61 218517 A (BIOMATERIAL UNIVERS), 29 September 1986 (1986-09-29)
- DATABASE WPI Section Ch, Week 198625 Derwent Publications Ltd., London, GB; Class A96, AN 1986-159567 XP002166689 & JP 61 093112 A (SEKISUI CHEM IND CO LTD) , 12 May 1986 (1986-05-12)
- DATABASE WPI Section Ch, Week 197528 Derwent Publications Ltd., London, GB; Class A96, AN 1975-46158W XP002166690 & DD 112 717 A (SCHILLER F), 5 May 1975 (1975-05-05)
- DATABASE WPI Section Ch, Week 198536 Derwent Publications Ltd., London, GB; Class A96, AN 1985-219157 XP002166691 & JP 58 105915 A (LION CORP), 24 June 1983 (1983-06-24)
- PATENT ABSTRACTS OF JAPAN vol. 013, no. 331 (C-622), 25 July 1989 (1989-07-25) & JP 01 110623 A (TEIKOKU SEIYAKU KK), 27 April 1989 (1989-04-27)
- N. KANIKKANNAN; ET AL.: "Transdermal delivery of indomethacin: I. Release profile of drug from polymeric patches." INDIAN DRUGS, vol. 30, no. 9, 1993, pages 441-445, XP008002545

## Description

### Field of the invention

The present invention refers to a single-layer film for active ingredients dermal and transdermal administration and to a method for the preparation of same.

### State of the art

In the last few years, active ingredients dermal and transdermal administration has been given a substantial boost thanks to the development of new arrangements - in particular dermal and transdermal sticking plasters - for active ingredients release to the skin.

Said sticking plasters usually consist of several layers of various materials, superimposed in the following sequence:
1. the **backing layer,** which is an essential element of sticking plasters found in commerce. It acts as a plaster "skeleton" and provides the suitable consistency for plaster handling and positioning on the skin. It is a transparent or opaque thin plastic film, usually occlusive to favour epidermis hydration. It must exhibit particularly good qualities of flexibility and resistance;
2. the **drug depot,** which is solid or semisolid or liquid and contains the active ingredient in the dispersed or dissolved state;
3. the **membrane for active ingredient controlled release:** once the sticking plaster has been applied, the membrane interposes between the drug depot and the skin and serves to control the active ingredient release rate;
4. the **adhesive layer,** which facilitates the plaster adhesion to the skin. It must secure the plaster contact with the skin surface and, at the same time, be permeable by the drug.

In addition to the aforementioned functional layers, the sticking plaster includes a protective layer consisting of a plastic sheet, coated with silicone polymers or fluoropolymers, which provide anti-adherent properties. Said layer protects the active ingredient and prevents unwanted adhesion during plaster handling and storage. The protective layer is removed immediately prior to the plaster use and, therefore, has no therapeutic function.

The sticking plasters found in commerce consist of all, or some, layers listed above. By way of example, the so-called "reservoir plasters" consist of all said layers, whereas other plasters, such as those referred to as "matrix", include all elements excepting the membrane. The simplest sticking plasters marketed today are the so-called "drug" in adhesive" ones, which consist of the backing and a drug/adhesive mixture exerting the double function of drug depot and adhesive layer. In "drug in adhesive" plasters, the drug is directly dispersed in the adhesive. Therefore, to our knowledge, in addition to the protective sheet, at least two layers are to be coupled in a dermal or transdermal sticking plaster.

Once applied, all sticking plasters exhibit a multi-layered structure in which the lower layer acts as an adhesive and the upper one as a support.

Dermal and transdermal sticking plasters are generally manufactured by lamination, whereby the single layers that already possess the required properties are superimposed one on top of the other. This method is rather complex and expensive as it requires preformed materials and elaborate procedures for layers coupling, and involves considerable material losses.

Furthermore, since the adhesives commonly used in plasters manufacture consist of water-insoluble polymers, the process must be carried out in the presence of organic solvents, e.g. ethyl acetate or toluene, which pose considerable safety problems.

Two-layered sticking plasters, i.e. the "drug in adhesive" ones, are prepared by simpler procedures, which envisage the spreading of the adhesive solution or viscous suspension on the preformed backing, followed by drying. However, also these sticking plasters suffer from the inconveniences caused by the presence of organic-based adhesives.

"EP0355536 describes a gel film which is swellable, but not soluble, in water, comprising at least one water-soluble polymer being anion-active at neutral pH, at least one water-soluble polymer being cation-active at neutral pH, and an additive which prevents the reaction of polymers of different charge, and which has a boiling temperature of 25°C to 120°C. Said additive can have basic character, e.g. primary, secondary and tertiary amines having boiling points between 25°C and 120°C, or can have acidic character, e.g. glacial acetic acid having a boiling point of 118-119°C. Said additive is thus an organic solvent, such as glacial acetic acid, an excellent polar protic solvent.

JP58078663 describes a film-forming base containing (a) 5-25 wt.% of a PVA, (b) 20-60 wt.% of monohydric lower alcohol, (c) 0.1-3.0 wt.% of ester gum. The solution obtained by dissolving specific PVA with ester gum in aq. EtOH solution having high EtOA concn. can maintain a homogeneous solution even when dissolving medicinal components, and by spreading and drying it on skin a film of good elasticity and adhesion to skin can be obtained. Thus the final film is formed *in-situ* directly on the skin.

JP61218517 describes a transdermal therapeutic article in the form of a film or sheet prepared by freezing an aqueous solution of polyvinyl alcohol containing pharmacologically active substances and pharmacologically acceptable additives at a temperature below -5°C, and then defreezing the frozen solution at a temperature of 0°C to 10°C for at least 10 hours. Additives are absorption enhancers selected from the group consisting of alcohols, esters, ketones, and alkyl sulfoxides, and/or tackifiers selected from the group consisting of poly(vinyl alcohol) with a low degree of saponification, gelatin, starch, hydroxypropyl cellulose, poly(ethylene oxide), poly(vinyl-N-pyrrolidone), acrylic acid copolymers, and sodium alginate.

US4210633 describes a film-forming formulation containing fluroandrenolide, that can be removed by peeling or washing. Said formulation is spread manually or with an applicator and the solvents present therein, including water, evaporate in 20-30 minutes, thus the final film is formed *in-situ* directly on the skin.

JP61093112 describes a traditional drug-in-adhesive plaster, which is a two-layer, self adhesive film and does not need film forming agents.

EP0184470 describes hydrogel pressure sensitive, adhesive composition having a water content of 27% to 40%.

GB1108837 describes a topically applicable local anaesthetic material comprising a sheet-like structure of water-soluble, film-forming material through which is uniformly distributed a local anaesthetic. Optionally, the film may be laminated or covered with a water-insoluble, impermeable or resistant backing material which also may be coated with an adhesive (on the side facing the film) and may also bear a removable covering layer to protect the film before use. No residual humidity is disclosed for said materials.

EP1110546 describes a method of preparing a water soluble film, comprising (a) preparing a solution comprising a film former, a water soluble plasticizer, a pharmaceutically active agent, and a solvent, preferably less than about 30% by weight of ethanol; (b) drying the solution at a temperature of from about 50 to about 100°C to form a film; and (c) curing the film at a temperature of from about 15 to about 60 °C and at a relative humidity of at least 30%. Kanikkannan et al. ('Transdermal delivery of indomethacin: I. release profile of drugs from polymeric patches' INDIAN DRUGS, VOL. vol. 30, no. 9, 1993, pages 441 - 445) describe traditional transdermal patches that require backing support and are self-adhesive."

As may be inferred from the above description, the technology for the manufacture of transdermal sticking plasters brings about considerable disadvantages, especially due to the manufacture complexity and to the use of organic solvents. Therefore, the need for an arrangement for active ingredients dermal and transdermal administration, manufactured by simple and little expensive procedures, which, furthermore, do not require organic solvents, is acknowledged.

### Summary

The Applicant has surprisingly found a new arrangement, in the form of a thin film, for active ingredients dermal and transdermal administration. The three elements that constitute the traditional sticking plaster, i.e. backing, drug depot, and adhesive layer, become indistinguishable and form a single element self-supporting. Said arrangement can be prepared by simple and little expensive procedures, which may use water-soluble polymers. Furthermore, being permeable by water, it may be easily tolerated and used for iontophoretic applications.

"The present invention thus concerns a non sticky in the dry state, self-supporting single-layer film having a residual humidity of between 4% and 20%, for dermal or transdermal administration of active ingredients, the surface of said film on contact with the skin regaining its adhesiveness in the presence of water, said film comprising at least:
a) an active ingredient,
b) a film-forming agent; and
c) a hydrophilic adhesive polymer,
wherein the film-forming agent/adhesive polymer ratio ranges from 2 to 7, and said film comprising no organic solvents."

### Description of the figures

Figure 1 is a bottom view (a) and a side view (b) of one of the possible embodiments of the film of the invention (I), supported by an antistick protective layer (II).
Figure 2 schematically illustrates the procedure for applying the film of the present invention to moistened skin.
Figure 3 shows the cumulative average amount of lidocaine (µg) per mg of stratum corneum after application of the single-layer film to moistened skin (I), of a commercial lidocaine formulation (II), of the single-layer film to non-moistened skin (III) or of the single-layer film to moistened skin with iontophoretic application (IV).
Figure 4 shows the average lidocaine distribution in the stratum corneum vs. the distance from the dermal surface: adhesive tapes 1-5, 6-10 and 11-15 include stratum corneum fragments localised at a gradually increasing distance from the surface.

### Detailed description of the invention

It is an object of the present invention to provide a single-layer film for active ingredients dermal and transdermal administration, comprising at least an active ingredient, a film-forming agent and a hydrophilic adhesive polymer.

The active ingredient may be in the dissolved or dispersed state.

The film of the present invention is useful for dermal or transdermal administration of any hydrophilic or lipophilic substance exerting a pharmacological or cosmetic action. Substances particularly suitable for administration through the film of the present invention are drugs for dermatologic use, e.g. topical anaesthetics, antimycotic drugs, antiinflammatory agents, cortisone-based drugs, antiviral agents, antineoplasia drugs, antihistamine drugs, antipsoriasis agents and antibiotics; drugs that may be administered by the transdermal way, e.g. nitroglycerin, sex hormones and nicotine; active ingredients for cosmetic use, e.g. keratolytics, keratoplastics, agents for the treatment of seborrhea, acne and depigmentation, disinfectants, and sebonormalisers.

The film-forming agent is preferably selected from the group consisting of ethylcellulose, acrylic and methacrylic polymers in an aqueous dispersion, and polyvinyl alcohol. According to the present invention by "acrylic and methacrylic polymers" is meant neutral acrylic and methacrylic polymers, i.e. acrylic and methacrylic polymers not having cationic or anionic charge, such as neutral copolymer based on ethyl acrylate and methyl methacrylate.

Preferably, the film-forming agent is polyvinyl alcohol having a molecular weight of 500 to 100,000 Da, especially of 49,000 to 72,000 Da. Said polyvinyl alcohol has a hydrolysis degree ranging preferably from 80% to 99%, especially from 85 to 89%. Preferably, the hydrophilic adhesive polymer is selected from the group consisting of polyvinylpyrrolidone, tragacanth, gum arabic, karaya. xanthan gum, guar gum, acrylic and methacrylic adhesives, carrageenan and rosin. Particularly preferred are polyaminomethacrylates, preferably Eudragit E100, and tragacanth. Water solutions of Eudragit E100, mixed with lauric acid, adipic acid and glycerin are available under the trademark Plastoid E 35 L, M and H from Röhm GmbH, Darmstadt, Germany.

In the film of the invention, particularly preferred is the combination of polyvinyl alcohol having a molecular weight of 500 to 100,000 Da, especially of 49,000 to 72,000 Da, as film-forming agent, with a polyaminomethacrylate, preferably Eudragit E100, or tragacanth, as a hydrophilic adhesive polymer. Preferably, said polyvinyl alcohol has a hydrolysis degree ranging from 80 to 99%, especially from 85 to 89%.

The single-layer film of the invention optionally comprises absorption promoters and/or humectants and/or plasticisers, e.g. glycerin, ethyl alcohol, propylene glycol, polyethylene glycol having a molecular weight ranging from 400 to 6,000, sorbitol, phospholipids, soybean lecithin, phosphatidyl choline, cholesterol, cyclodextrins, isopropyl myristate, oleic acid, polysorbate 80, diethylene glycol monoethyl ether (Transcutol, Gattefossée, France).

Preferably, the film of the present invention is 20 to 500 µm thick.

It is a further object of the present invention to provide a process for the preparation of the aforesaid single-layer film supported by an antistick protective sheet, which comprises the following steps:
a) preparing a water solution of the film-forming agent;
b) adding the solution of step a) to a solution of the hydrophilic adhesive polymer ", to have a film-forming agent/adhesive polymer ratio ranging from 2 to 7";
c) adding one active ingredient at least, in the form of water solution or micronised particles or emulsion;
d) spreading the mixture obtained in step c) as a thin layer, preferably 50 to 1,000 µm thick, on an antistick sheet of plastic material or aluminium or paper coated with silicone or fluoropolymers (e.g. available from 3M, USA, or from Rexam Release, USA);
e) drying the layer obtained in step d) to residual humidity of 4% to 20%.

Drying is carried out by conventional methods, e.g. by oven or infra-red rays drying.

The single-layer film obtained, supported by an antistick protective sheet, may be opportunely divided into portions having the shape and surface suitable for the various therapeutic applications and may be suitably packaged, ready for use, in sterile air-tight packages.

Preferably, the mixture obtained in step c) consists of 0.1% to 20% active ingredient, 5% to 40% (w/w) film-forming agent, 1% to 15% (w/w) adhesive polymer, and 50% to 85% water.

In step c) the adhesive/film-forming mixture is optionally added not only with the active ingredient but also with 0.5% to 20% (w/w) of one or more substances acting as absorption promoters and/or humectants and/or plasticisers.

Preferably, the mixture of step c) - to be adequately smeared - should have a viscosity of 1,000 to 50,000 mPa.s, measured at a 10 rpm flow gradient by a rotary viscosimeter, Viscostar (Fungilab, France) with head TR11.

Once step e) has been completed, the drug/adhesive/film-forming agent is thinned down in consistency; the film surface exposed to the air loses most of its adhesiveness.

The present invention substantially differs from the transdermal arrangements already known not only in the number of layers but also because the protective sheet does not cover the adhesive surface, but covers the opposite surface.

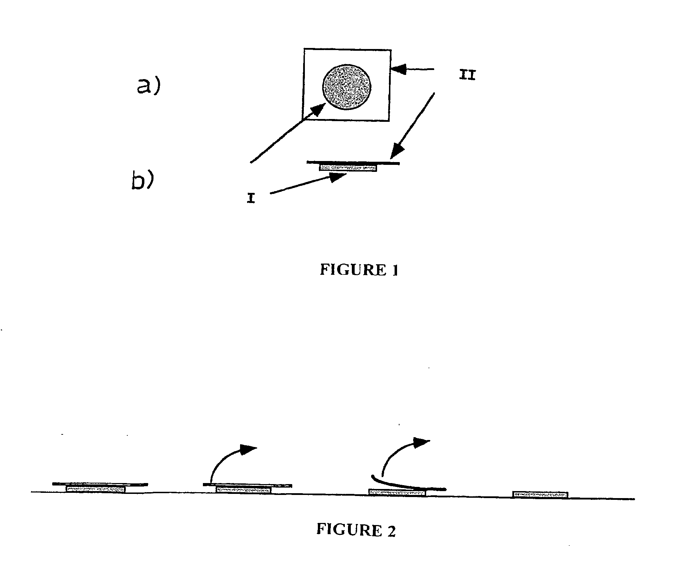

On application, the surface exposed to the air is maintained on the water- or saliva-moistened skin by applying a slight pressure for few seconds. Thanks to the presence of water, the surface in contact with the skin regains its adhesiveness, and by removing the protective sheet, the drug/adhesive/film-forming layer is transferred onto the skin as a transparent film with a non-sticky surface (Figure 2), which adheres to the skin firmly and integrally for at least 24 hrs. Adhesiveness is secured by the micro-moisture that forms, as a result of perspiration, between the skin and the film. Conversely, the moisture of the upper layer, initially present after the protective film removal, dries out by exposure to the air.

Since the film of the present invention conducts electricity, it can be advantageously used for active ingredients transdermal administration by iontophoretic applications, whereby the quantity of active ingredient that crosses the skin and reaches the systemic circulation increases.

The film of the present invention offers many advantages over the semisolid formulations or sticking plasters currently used for active ingredients dermal and transdermal administration.

In particular, compared with traditional dermal and transdermal sticking plasters, the single-layer film offers the advantages listed below:
1. it can be prepared by a simple and no expensive procedure, which, furthermore, does not require organic solvents;
2. it is thin and very flexible and, therefore, perfectly adapts itself to the skin wrinkles and lines; hence, the film surface in contact with the skin and, consequently, the active ingredient release increase considerably;
3. it can be easily handled as it is non-sticky in the dry state;
4. it is permeable by water with the result that it does not cause the occlusive effect typical of plasters;
5. it conducts electricity and, therefore, can be used for iontophoretic applications. As concerns iontophoresis, the film of the invention offers the following advantages:

1. the active ingredient keeps in contact with the skin even once the iontophoretic application has been completed;
2. it allows a greater adherence to the skin during and after iontophoretic application;
3. it simplifies iontophoresis procedures and makes them fit for outpatient use. The following examples are given further to illustrate the present invention.

### Example 1

### Preparation of a single-layer film containing lidocaine chlorhydrate

Polyvinyl alcohol (13.02 g) having a molecular weight of 72,000 Da and a hydrolysis degree of 86% to 89% was dispersed in water (49 ml), previously heated to 80°C. The resulting mixture was stirred to complete dissolution. Separately, for adhesive preparation, water (18.15 ml), previously heated to 78°C to 82°C, was added with Eudragit E100 (4.3 g), lauric acid (2.48 g) and adipic acid (0.48 g). The mixture was stirred at a constant temperature for approx. 30 min, cooled to 60°C, and added with glycerin (1.57 g). In a separate vessel, lidocaine chlorhydrate (2 g) was dissolved in water (5 ml). The polyvinyl alcohol solution was then added, in the order, with the adhesive solution, lidocaine solution and glycerin (4 g).

The mass obtained was spread, in the form of a thin film (250 µm thick), on a silicone-coated paper sheet ("liner") with doctor blade (BYK-Gardner, Silver Spring, USA). The resulting product was fed to an air-circulated oven at 60°C for a period of 30 min. Once the treatment was complete, round portions (approx. 7 cm² each) were cut from the coated strip.

The single-layer film obtained was 40µm thick and had a lidocaine content of 2 mg/portion, i.e. 0.3 mg/cm² or 74 mg/cm³/portion.

### Example 2

### Preparation of a single-layer film containing acyclovir

Polyvinyl alcohol (18.6 g) having a molecular weight of 49,000 Da and a hydrolysis degree of 86% to 89% was dispersed in water (44 ml), previously heated to 80°C. The resulting mixture was stirred to complete dissolution. Separately, for adhesive preparation, water (18.2 ml), previously heated to 78°C-82°C, was added with Eudragit E100 (4.3 g), lauric acid (2.48 g) and adipic acid (0.48 g). The mixture was stirred for approx. 30 min at constant temperature, cooled to 60°C, and added with glycerin (0.27 g). In a separate vessel, acyclovir (1.5 g) was dispersed in glycerin (4 ml). The polyvinyl alcohol solution was then added, in the order, with the adhesive solution, an acyclovir dispersion and 6.17 g of a 70% sorbitol solution.

In this case, the active ingredient (acyclovir) was dispersed in the form of particles in the adhesive/film-forming mixture.

The mass obtained was spread, in the form of a thin film (250 µm thick), on a silicone-coated sheet of polymeric material ("liner") with doctor blade (BYK-Gardner, Silver Spring, USA). The resulting product was fed to an air-circulated oven at 60°C for a period of 30 min. Once the treatment was complete, round portions (approx. 7 cm² each) were cut from the coated strip.

The single-layer film obtained was 40µm thick.

### Example 3

### Preparation of a single-layer film containing 5-methoxypsoralen

Polyvinyl alcohol (18.6 g) having a molecular weight of 49,000 Da and a hydrolysis degree of 86% to 89% was dispersed in water (44 ml). The resulting mixture was stirred to complete dissolution. Separately, for adhesive preparation, water (19.33 ml), previously heated to 78°C-82°C, was added with Eudragit E100 (4.3 g), lauric acid (2.48 g) and adipic acid (0.48 g). The mixture was stirred for approx. 30 min at a constant temperature, cooled to 60°C, and added with glycerin (0.27 g). In a separate vessel, 5-methoxypsoralen (0.01 g), cholesterol (0.08 g) and lecithin (0.07 g) were dissolved in ethanol (2.72 g) and isopropyl myristate (0.93 g). The solution was added with water (3 g) to form an emulsion. The polyvinyl alcohol solution was then added, in the order, with the adhesive solution, the drug-containing emulsion and glycerin (3.73 g).

The mass obtained was spread, in the form of a thin film (300 µm thick), on a silicone-coated sheet ("liner") with doctor blade (BYK-Gardner, Silver Spring, USA). The resulting product was fed to an air-circulated oven at 60°C for a period of 30 min. Once the treatment was complete, round portions (approx. 7 cm² each) were cut from the coated strip.

The single-layer film obtained, 40µm thick, had an active ingredient content of 10 µg/portion.

### Example 4

### Preparation of a single-layer film containing ibuprofen lysine

Polyvinyl alcohol (13.02 g) having a molecular weight of 72,000 Da and a hydrolysis degree of 86% to 89% was dispersed in water (49 ml) previously heated to 80°C. The resulting mixture was stirred to complete dissolution. Separately, for adhesive preparation, water (25 ml), previously heated to 80°C, was added with tragacanth (2.08 g). The mixture was stirred to complete dissolution. In a separate vessel, ibuprofen lysine (3 g) was dissolved in water (2 ml). The polyvinyl alcohol solution was then added, in the order, with the adhesive solution, an ibuprofen lysine solution and 5.9 g of a 70% sorbitol solution.

The mass obtained was spread, in the form of a thin film (300 µm thick), on a silicone-coated sheet of polymeric material ("liner") with doctor blade (BYK-Gardner, Silver Spring, USA). The resulting product was fed to an air-circulated oven at 60°C for a period of 30 min. Once the treatment was complete, round portions (approx. 7 cm² each) were cut from the coated strip.

The single-layer film obtained was 40µm thick.

### Example 5

### Assessment of active ingredients release In vivo

The *in vivo* active ingredient release from the film prepared as per Example 1 was evaluated on volunteers, 24 to 26 years of age, using the tape stripping technique, proposed by the US FDA for the determination of the bioavailability/bioequivalence of topical formulations (US FDA, Topical dermatological drug products, NDAs and ANDAs - *In vivo* bioavailability, bioequivalence, *in vitro* release and associated studies, CDAR, 1998).

This technique is based on the removal of small portions of stratum corneum by repeated applications of the adhesive tape to the skin and successive extraction and analysis of the active ingredient contained therein.

To go into details, single-layer film portions obtained as per Example 1 were applied to the volunteers' forearm moistened skin and maintained there, with or without iontophoretic applications, for a period of 30 min. After said period, they were removed and tape stripping was carried out. In case of application in the presence of iontophoresis, an electrocardiography electrode connected to the positive pole of a constant-intensity d.c. generator, was attached to the film. A current density of 0.5 mA/cm² was applied for 30 min.

For purpose of comparison, a commercial formulation consisting of 2.5% lidocaine chlorhydrate gel, in an amount of 15 mg/cm² (corresponding to 0.3 mg/cm² lidocaine) was applied to a different part of the same forearm for 30 min. After said period, the formulation was removed with moistened cotton-wool. Still for purpose of comparison, the film as per Example 1 was applied to non-moistened skin for 30 min. In both cases, tape stripping was performed. To go into details, the adhesive tape was consecutively applied 15 times to the same skin area that had been in contact with the film or with the lidocaine-containing gel. Each adhesive tape was weighed before and after application: the quantity of stratum corneum removed every time was determined. The adhesive tapes taken from a single volunteer were collected, in sequence, five at a time, in a test tube. Therefore, three samples per volunteer were obtained for each type of application, i.e. the first consisted of adhesive tapes 1-5, the second of adhesive tapes 6-10, and the third of adhesive tapes 11-15, including stratum corneum fragments localised at a different distance from the surface. The lidocaine present in each sample was then extracted with an eluent (3 ml) and analysed by high-performance liquid chromatograhy, using 300 x 3.9 mm µ-Bondapak C-18 (Waters) column (Millipore, Milford, United States). The eluent used was a mixture of acetonitrile (14 parts) and 0.05 M potassium phosphate (86 parts), pumped at a flow rate of 1 milliliter per minute and monitored by spectrophotometer at 216 nm. The detected amount of lidocaine was normalised in respect of the amount of stratum corneum contained in each sample of adhesive tape.

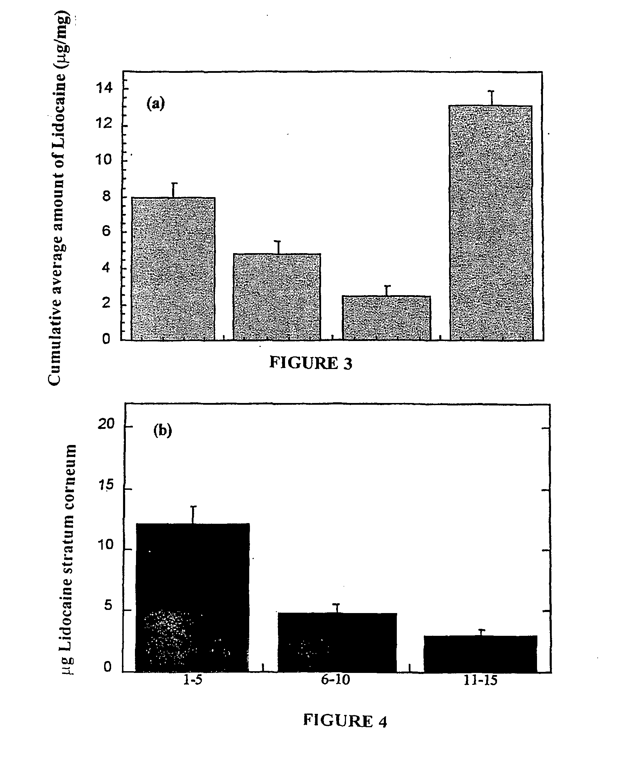

The results obtained are shown in Figures 3 and 4.

Figure 3 shows the cumulative average amount of lidocaine per mg stratum corneum detected after application of the single-layer film to moistened skin (I); of a commercial formulation of lidocaine chlorhydrate (Luan^{®}) (II); of the single-layer film to non-moistened skin (III); of single-layer film on moistened skin with iontophoretic application (IV). The data obtained prove that the single-layer film of the invention provides much higher active ingredient tissual concentrations than the traditional formulations and that said concentrations may be further increased by iontophoretic application. Furthermore, to adhere to the skin and release the drug appropriately, the film must be applied to moistened skin.

Figure 4 shows the average distribution of lidocaine in the stratum corneum vs. the skin distance. As may be seen from the Figure, although lidocaine is especially present in the stratum corneum upper layers, non-negligible amounts also pass into the deeper layers.

## Claims

1. A non sticky in the dry state, self-supporting single-layer film having a residual humidity of between 4% and 20%, for dermal or transdermal administration of active ingredients, the surface of said film on contact with the skin regaining its adhesiveness in the presence of water, said film comprising at least:
a) an active ingredient,
b) a film-forming agent; and
c) a hydrophilic adhesive polymer,
wherein the film-forming agent/adhesive polymer ratio ranges from 2 to 7, and said film comprising no organic solvents.

2. The film as claimed in claim 1, wherein said active ingredient is in the dissolved or dispersed state.

3. The film as claimed in claims 1 or 2, wherein said film-forming agent is selected from the group consisting of ethylcellulose, acrylic and methacrylic polymers in an aqueous dispersion, and polyvinyl alcohol.

4. The film as claimed in claim 3, wherein said film-forming agent is polyvinyl alcohol having a molecular weight ranging from 500 to 100,000 Da.

5. The film as claimed in claim 4, wherein said polyvinyl alcohol has a molecular weight ranging from 49,000 to 72,000 Da.

6. The film as claimed in claim 4 or 5, wherein said polyvinyl alcohol has a hydrolysis degree ranging from 80% to 99%.

7. The film as claimed in claim 6, wherein said polyvinyl alcohol has a hydrolysis degree ranging from 85% to 89%.

8. The film as claimed in anyone of claims 1 to 7, wherein said hydrophilic adhesive polymer is selected from the group consisting of polyvinylpyrrolidone, tragacanth, gum arabic, karaya, xanthan gum, guar gum, acrylic and methacrylic adhesives, carrageenan and rosin.

9. The film as claimed in claim 8, wherein said hydrophilic adhesive polymer is a polyaminomethacrylate or tragacanth.

10. The film as claimed in claim 9, wherein said polyaminomethacrylate is Eudragit E100.

11. The film as claimed in claim 1, wherein said film-forming agent is polyvinyl alcohol having a molecular weight ranging from 500 to 100,000 Da and said hydrophilic adhesive polymer is a polyaminomethacrylate or tragacanth.

12. The film as claimed in claim 11, wherein said polyaminomethacrylate is Eudragit E100.

13. The film as claimed in claim 11, wherein said polyvinyl alcohol has a molecular weight ranging from 49,000 to 72,000 Da.

14. The film as claimed in claim 11, wherein said polyvinyl alcohol has a hydrolysis degree ranging from 80% to 99%.

15. The film as claimed in claim 14, wherein said polyvinyl alcohol has a hydrolysis degree ranging from 85% to 89%.

16. The film as claimed in anyone of claims 1 to 15 comprising at least a substance acting as an absorption promoter and/or humectant and/or plasticiser.

17. The film as claimed in claim 16, wherein said substance is selected from the group consisting of glycerin, ethyl alcohol, propylene glycol, polyethylene glycol having a molecular weight ranging from 400 to 6,000, sorbitol, phospholipids, soybean lecithin, phosphatidyl choline, cholesterol, cyclodextrins, isopropyl myristate, oleic acid, polysorbate 80, diethylene glycol monoethyl ether.

18. The film as claimed in anyone of claims 1 to 17 having a thickness of 20 to 500 µm.

19. The film as claimed in anyone of claims 1 to 18 supported by a removable antistick protective sheet.

20. The film as claimed in anyone of claims 1-19 for transdermal iontophoretic applications.

21. Process for the preparation of a single-layer film as claimed in anyone of claims 1 to 19, comprising the following steps:
a) preparing a water solution of the film-forming agent;
b) adding the solution of step a) to a solution of the hydrophilic adhesive polymer, to have a film-forming agent/adhesive polymer ratio ranging from 2 to 7;
c) adding at least one active ingredient;
d) spreading the mixture obtained in step c) as a thin layer, on an antistick sheet of plastic material or aluminium or paper coated with silicone or fluoropolymers;
e) drying the layer obtained in step d) to residual humidity of 4% to 20%.

22. The process as claimed in claim 21, wherein the layer obtained in step d) is 50 µm to 1,000 µm thick.

23. The process as claimed in claim 21 or 22, wherein the mixture obtained in step c) has a viscosity ranging from 1,000 to 50,000 mPa.s.

24. The process as claimed in anyone of claims 21 to 23, wherein the mixture obtained in step c) consists of 0.1 % to 20% active ingredient, 5% to 40% (w/w) film-forming agent, 1% to 15% (w/w) adhesive polymer and 50% to 85% water.

25. The process as claimed in claim 24, wherein the film-forming agent/adhesive polymer ratio in said mixture ranges from 2 to 7.

26. The process as claimed in anyone of claims 21 to 25, wherein in said step c) said active ingredient is dissolved in the solution comprising the film-forming agent and the adhesive polymer.

27. The process as claimed in anyone of claims 21 to 25, wherein in said step c) said active ingredient is dispersed in the solution comprising the film-forming agent and the adhesive polymer in the form of micronised particles or of emulsion.

28. The process as claimed in anyone of claims 21 to 27, wherein in said step c) the solution comprising the film-forming agent and the adhesive polymer is added with 0.5% to 20% (w/w) at least of a substance acting as an absorption promoter and/or humectant and/or plasticiser.

## Patentansprüche

1. Im Trockenzustand nicht-klebriger, selbsttragender Einzelschichtfilm mit einer Restfeuchtigkeit zwischen 4% und 20% zur dermalen oder transdermalen Verabreichung von aktiven Bestandteilen, wobei die Oberfläche von dem Film in Kontakt mit der Haut in der Gegenwart von Wasser deren Klebrigkeit wiedererlangt, wobei der Film wenigstens umfasst:
a) einen aktiven Bestandteil,
b) ein filmbildendes Mittel und
c) ein hydrophiles Klebstoffpolymer,
wobei das Verhältnis zwischen dem filmbildenden Mittel/Klebstoffpolymer in einem Bereich zwischen 2 und 7 liegt und der Film keine organischen Lösungsmittel enthält.

2. Film nach Anspruch 1, wobei der aktive Bestandteil in dem gelösten oder dispergierten Zustand vorliegt.

3. Film nach Anspruch 1 oder 2, wobei das filmbildende Mittel aus der Gruppe ausgewählt ist, welche aus Ethylcellulose, Acryl- und Methacrylpolymeren in einer wässrigen Dispersion und Polyvinylalkohol besteht.

4. Film nach Anspruch 3, wobei das filmbildende Mittel ein Polyvinylalkohol mit einem Molekulargewicht in einem Bereich von 500 bis 100.000 Da ist.

5. Film nach Anspruch 4, wobei der Vinylalkohol ein Molekulargewicht zwischen 49.000 und 72.000 Da aufweist.

6. Film nach Anspruch 4 oder 5, wobei der Polyvinylalkohol einen Hydrolysegrad in einem Bereich zwischen 80% und 99% aufweist.

7. Film nach Anspruch 6, wobei der Polyvinylalkohol einen Hydrolysegrad in einem Bereich von 85% und 89% aufweist.

8. Film nach einem der Ansprüche 1 bis 7, wobei das hydrophile Klebstoffpolymer aus der Gruppe ausgewählt ist, welche aus Polyvinylpyrrolidon, Traganth, Gummi arabicum, Karaya, Xanthangummi, Guargummi, Acryl- und Methacrylklebstoffen, Carrageen und Harz besteht.

9. Film nach Anspruch 8, wobei das hydrophile Klebstoffpolymer ein Polyaminomethacrylat oder Traganth ist.

10. Film nach Anspruch 9, wobei das Polyaminomethacrylat Eudragit E100 ist.

11. Film nach Anspruch 1, wobei das filmbildende Mittel Polyvinylalkohol mit einem Molekulargewicht in einem Bereich zwischen 500 und 100.000 Da ist und das hydrophile Klebstoffpolymer ein Polyaminomethacryl oder Traganth ist.

12. Film nach Anspruch 11, wobei das Polyaminomethacrylat Eudragit E100 ist.

13. Film nach Anspruch 11, wobei der Polyvinylalkohol ein Molekulargewicht in einem Bereich zwischen 40.000 und 72.000 Da aufweist.

14. Film nach Anspruch 11, wobei der Polyvinylalkohol einen Hydrolysegrad in einem Bereich von 80% bis 99% aufweist.

15. Film nach Anspruch 14, wobei der Polyvinylalkohol einen Hydrolysegrad in einem Bereich von 85% und 89% aufweist.

16. Film nach einem der Ansprüche 1 bis 15 enthaltend wenigstens eine Verbindung, welche als ein Absorptionspromoter und/oder Feuchthaltemittel und/oder Weichmacher wirkt.

17. Film nach Anspruch 16, wobei die Verbindung aus der Gruppe ausgewählt ist, welche aus Glycerin, Ethylalkohol, Propylenglycol, Polyethylenglycol mit einem Molekulargewicht in einem Bereich zwischen 400 und 6.000, Sorbit, Phosphoripiden, Sojabohnenlecithin, Phospatidylcholin, Cholesterin, Cyclodextrinen, Isopropylmyristat, Ölsäure, Polysorbat 80, Diethylenglycolmonoethylether besteht.

18. Film nach einem der Ansprüche 1 bis 17 mit einer Dicke von 20 bis 500 µm.

19. Film nach einem der Ansprüche 1 bis 18, welcher auf einer entfernbaren antiklebenden Schutzfolie getragen ist.

20. Film nach einem der Ansprüche 1 bis 19 für transdermale iontophoretische Anwendungen.

21. Verfahren zum Herstellen eines Einzelschichtfilms nach einem der Ansprüche 1 bis 19, welches die nachfolgenden Schritte umfasst:
a) Herstellen einer Wasserlösung des filmbildenden Mittels,
b) Zugabe der Lösung aus dem Schritt a) zu einer Lösung des hydrophilen Klebstoffpolymers, um ein filmbildendes Mittel/Klebstoffpolymer-Verhältnis in einem Bereich von 2 bis 7 zu erhalten,
c) Zugabe wenigstens eines aktiven Bestandteils,
d) Ausbreiten der in dem Schritt c) erhaltenen Mischung als einen dünnen Film auf ein nicht-klebendes Blatt aus Kunststoffmaterial oder Aluminium oder mit Silikon oder Fluorpolymeren beschichtetem Papier,
e) Trocknen der in dem Schritt d) erhaltenen Schicht auf eine restliche Feuchtigkeit von 4% bis 20%.

22. Verfahren nach Anspruch 21, wobei die in dem Schritt d) erhaltene Schicht 50 µm bis 1.000 µm dick ist.

23. Verfahren nach Anspruch 21 oder 22, wobei die in dem Schritt c) erhaltene Mischung eine Viskosität in einem Bereich von 1.000 bis 50.000 mPa s aufweist.

24. Verfahren nach einem der Ansprüche 21 bis 23, wobei die in dem Schritt c) erhaltene Mischung aus 0,1% bis 20% aktivem Bestandteil, 5% bis 40% (Gew./Gew.) filmbildendem Mittel, 1% bis 15% (Gew./Gew.) Klebstoffpolymer und 50% bis 85% Wasser besteht.

25. Verfahren nach Anspruch 24, wobei das Verhältnis aus filmbildendem Mittel/Klebstoffpolymer in der Mischung in einem Bereich zwischen 2 und 7 liegt.

26. Verfahren nach einem der Ansprüche 21 bis 25, wobei der aktive Bestandteil in dem Schritt c) in der Lösung, welche das filmbildende Mittel und das Klebstoffpolymer enthält, gelöst wird.

27. Verfahren nach einem der Ansprüche 21 bis 25, wobei der aktive Bestandteil in dem Schritt c) in der Lösung, welche das filmbildende Mittel und das Klebstoffpolymer enthält, in der Form von mikronisierten Partikeln oder einer Emulsion dispergiert wird.

28. Verfahren nach einem der Ansprüche 21 bis 27, wobei die das filmbildende Mittel und das Klebstoffpolymer enthaltende Lösung in dem Schritt c) mit 0,5% bis 20% (Gew./Gew.) wenigstens einer Substanz, welche als ein Absorptionspromoter und/oder Feuchthaltemittel und/oder Weichmacher agiert, zugegeben wird.

## Revendications

1. Film à couche unique autoporteur, non collant à l'état sec, présentant une humidité résiduelle comprise entre 4 % et 20 %, pour l'administration cutanée ou transcutanée de principes actifs, la surface dudit film, au contact de la peau, récupérant son adhésivité en présence d'eau, ledit film comprenant au moins :
a) un principe actif,
b) un agent filmogène ; et
c) un polymère adhésif hydrophile,
dans lequel le rapport agent filmogène/polymère adhésif va de 2 à 7, et ledit film ne comprend pas de solvant organique.

2. Film selon la revendication 1, dans lequel ledit principe actif est à l'état dissous ou dispersé.

3. Film selon la revendication 1 ou 2, dans lequel ledit agent filmogène est choisi dans le groupe consistant en l'éthylcellulose, des polymères acryliques et méthacryliques dans une dispersion aqueuse, et l'alcool polyvinylique.

4. Film selon la revendication 3, dans lequel ledit agent filmogène est l'alcool polyvinylique présentant une masse moléculaire allant de 500 à 100 000 Da.

5. Film selon la revendication 4, dans lequel ledit alcool polyvinylique présente une masse moléculaire allant de 49 000 à 72 000 Da.

6. Film selon la revendication 4 ou 5, dans lequel ledit alcool polyvinylique présente un degré d'hydrolyse allant de 80 % à 99 %.

7. Film selon la revendication 6, dans lequel ledit alcool polyvinylique présente un degré d'hydrolyse allant de 85 % à 89 %.

8. Film selon l'une quelconque des revendications 1 à 7, dans lequel ledit polymère adhésif hydrophile est choisi dans le groupe consistant en la polyvinylpyrrolidone, la gomme adragante, la gomme arabique, la gomme de karaya, la gomme de xanthane, la gomme de guar, les adhésifs acryliques et méthacryliques, la carraghénane et la colophane.

9. Film selon la revendication 8, dans lequel ledit polymère adhésif hydrophile est un polyaminométhacrylate ou la gomme adragante.

10. Film selon la revendication 9, dans lequel ledit polyaminométhacrylate est Eudragit E100.

11. Film selon la revendication 1, dans lequel ledit agent filmogène est l'alcool polyvinylique présentant une masse moléculaire allant de 500 à 100 000 Da et ledit polymère adhésif hydrophile est un polyaminométhacrylate ou la gomme adragante.

12. Film selon la revendication 11, dans lequel ledit polyaminométhacrylate est Eudragit E100.

13. Film selon la revendication 11, dans lequel ledit alcool polyvinylique présente une masse moléculaire allant de 49 000 à 72 000 Da.

14. Film selon la revendication 11, dans lequel ledit alcool polyvinylique présente un degré d'hydrolyse allant de 80 % à 99 %.

15. Film selon la revendication 14, dans lequel ledit alcool polyvinylique présente un degré d'hydrolyse allant de 85 % à 89 %.

16. Film selon l'une quelconque des revendications 1 à 15, comprenant au moins une substance agissant comme un promoteur d'absorption et/ou humidifiant et/ou plastifiant.

17. Film selon la revendication 16, dans lequel ladite substance est choisie dans le groupe consistant en la glycérine, l'alcool éthylique, le propylèneglycol, le polyéthylèneglycol présentant une masse moléculaire allant de 400 à 6 000, le sorbitol, les phospholipides, la lécithine de soja, la phosphatidylcholine, le cholestérol, les cyclodextrines, le myristate d'isopropyle, l'acide oléique, le polysorbate 80, l'éther monoéthylique du diéthylèneglycol.

18. Film selon l'une quelconque des revendications 1 à 17 ayant une épaisseur de 20 à 500 µm.

19. Film selon l'une quelconque des revendications 1 à 18 soutenu par une feuille de protection antiadhésive amovible.

20. Film selon l'une quelconque des revendications 1 à 19 pour des applications transcutanées par ionophorèse.

21. Procédé pour la préparation d'un film à couche unique selon l'une quelconque des revendications 1 à 19, comprenant les étapes suivantes :
a) la préparation d'une solution aqueuse de l'agent filmogène ;
b) l'ajout de la solution de l'étape a) à une solution du polymère adhésif hydrophile, pour avoir un rapport agent filmogène/polymère adhésif allant de 2 à 7 ;
c) l'ajout d'au moins un principe actif ;
d) l'étalement du mélange obtenu à l'étape c) en une couche fine, sur un feuille antiadhésive de matière plastique ou d'aluminium ou de papier revêtu de silicone ou de polymères fluorés ;
e) le séchage de la couche obtenue à l'étape d) jusqu'à une humidité résiduelle de 4 % à 20 %.

22. Procédé selon la revendication 21, dans lequel la couche obtenue à l'étape d) a une épaisseur de 50 µm à 1 000 µm.

23. Procédé selon la revendication 21 ou 22, dans lequel le mélange obtenu à l'étape c) présente une viscosité allant de 1 000 à 50 000 mPa.s.

24. Procédé selon l'une quelconque des revendications 21 à 23, dans lequel le mélange obtenu à l'étape c) consiste en 0,1 % à 20 % de principe actif, 5 % à 40 % (p/p) d'agent filmogène, 1 % à 15 % (p/p) de polymère adhésif et 50 % à 85 % d'eau.

25. Procédé selon la revendication 24, dans lequel le rapport agent filmogène/polymère adhésif dans ledit mélange va de 2 à 7.

26. Procédé selon l'une quelconque des revendications 21 à 25, dans lequel, à ladite étape c), ledit principe actif est dissous dans la solution comprenant l'agent filmogène et le polymère adhésif.

27. Procédé selon l'une quelconque des revendications 21 à 25, dans lequel, à ladite étape c), ledit principe actif est dispersé dans la solution comprenant l'agent filmogène et le polymère adhésif sous forme de particules micronisées ou d'émulsion.

28. Procédé selon l'une quelconque des revendications 21 à 27, dans lequel, à ladite étape c), la solution comprenant l'agent filmogène et le polymère adhésif est ajoutée avec 0,5 % à 20 % (p/p) d'au moins une substance agissant comme un promoteur d'absorption et/ou humidifiant et/ou plastifiant.
